# EUROPEAN PATENT APPLICATION

(11) **EP 2 505 663 A1**
(43) Date of publication of application: **03.10.2012**
(21) Application number: 11160387.4
(22) Date of filing: 30.03.2011
(51) Int. Cl.: C12Q 1/68

(54) **A method to identify asymptomatic high-risk individuals with early stage lung cancer by means of detecting miRNAs in biologic fluids**

(71) Applicant: IFOM Fondazione Istituto Firc di Oncologia Molecolare, 20139 Milano (IT); Instituto Europeo di Oncologia S.r.l., 20121 Milano (IT); Universita' Degli Studi Di Milano, 20122 Milano (IT)
(72) Inventor: Di Fiore, Pier Paolo, 20139 Milano (IT); Bianchi, Fabrizio, 20139 Milano (IT); Nicassio, Francesco, 20139 Milano (IT); Marzi, Matteo Jacopo Luca Nicolo', 20139 Milano (IT)
(74) Representative: Capasso, Olga

(57) **Abstract**

A diagnostic and/or prognostic model of at least 5 serum miRNAs selected among a group of 34 miRNAs that identifies subjects with early stage lung cancer, namely non-small cell lung carcinomas (NSCLCs), in a population of asymptomatic high-risk individuals, with 80% accuracy. The model could also distinguish between benign and malignant lesions and is sensitive enough to capture the disease onset. In addition, the model could also diagnose or confirm a diagnosis of lung cancer in symptomatic patients, and monitor the lung tumor status after treatment with surgery and/or chemotherapy and/or radiotherapy in a subject with such lung cancer.

## Description

### TECHNICAL FIELD

The invention refers to methods and means to diagnose and prognose lung cancer. More in particular the invention refers to a biological fluid miRNA test to detect early stage lung cancer in asymptomatic subjects.

### BACKGROUND

Lung cancer, in particular Non-small cell lung carcinoma (NSCLC) is the first cause of cancer mortality worldwide², largely due to the lack of effective tools for early diagnosis. Subjects diagnosed with early stage disease (stage I) have a reasonably favorable prognosis (60-80% survival at 5 years³). Unfortunately, only 20-25% of all NSCLCs are diagnosed early^{4,5}. Tobacco exposure is the major risk factor for lung cancer⁶. Even in former smokers, the tobacco-associated risk remains high for a long time⁷. Thus, effective tools for the early detection of NSCLC, especially in high-risk individuals, are critically needed. Low dose spiral computed tomography (LD-CT) has been tested in prospective observational trials, and has proven to be an effective detection method for small nodules (even < 5 mm) and for the diagnosis of asymptomatic lung cancers, most of which (67-85%) are at stage I^{1,8-10}. There is debate over whether annual LD-CT screening reduces diagnoses of advanced stage disease (stages III-IV) and lung cancer deaths¹¹⁻¹³, and the possibility has been raised that LD-CT screening may lead to over-diagnosis and over-treatment of biologically indolent cancers which would rarely reach clinical relevance or pose a health hazard for subjects^{14,15}. However, the effectiveness of LD-CT has recently been confirmed, as a large randomized trial (The National Lung Screening Trial¹⁶) was prematurely stopped on the basis that "the accumulated data now provide a statistically convincing answer to the study's primary question"
(http://www.cancer.gov/newscenter/pressreleases/NLSTresuItsRelease).

If early diagnosis is indeed an effective method to reduce lung cancer deaths, then the development of diagnostic blood tests becomes of paramount relevance for the execution of effective population screenings. The detection of specific circulating microRNAs (miRNAs) might serve this purpose. Indeed there is evidence to support the idea that circulating microRNAs with diagnostic potential exist for almost every type of malignant¹⁷⁻²⁶ and non-malignant disease²⁷⁻³¹, including lung cancer³²⁻³⁴.

### PRIOR ART

Some prior art disclose the use of detecting miRNAs to diagnose symptomatic cancer, also lung cancer. However no prior art document discloses a method that effectively work at early stage of disease when subjects are still asymptomatic.

Prior art documents comprise:
- Lu J et al Nature, 2005 Jun 9;435(7043):834-8 highlighted the potential of miRNA profiling in cancer diagnosis.
- Chen X, et al. Cell Research Cell Res. 2008 Oct;18(10):997-1006 concluded that serum miRNAs can serve as potential biomarkers for the detection of various cancers and other diseases.
- Bishop Ja, et al. Clinical Cancer Research, Clin Cancer Res. 2010 Jan 15;16(2):610-9 stated that miRNA profiling is a highly reliable strategy for classifying NSCLCs. The assay was based on RT-PCR from biopsies/aspirates and focused on hsa-miR-205.
- Gilad S, et al. Plos One PLoS One. 2008 Sep 5;3(9):e3148 highlighted the medically relevant potential of determining microRNA levels in serum and other body fluids.
- Shen et al. Lab Invest. 2010 Nov 29.identified 12 expression altered miRNAs in plasma of lung NSCLC affected subjects.
- Keller et al, BMC Cancer, 2009 Oct 6;9:353 detected 27 miRNAs deregulated in blood cells of lung cancer subjects (NSCLC); by using 24 of them, they were able to distinguish between blood cells samples of tumor patients and controls. All of above prior art refers to lung cancer patients, with no disclosure of a method to early diagnose of asymptomatic individuals.
- Yu S-L et al. Cancer Cell 2008 Jan;13(1):48-57 investigated whether microRNA expression profiles could predict clinical outcome of NSCLC patients. Using real-time RT-PCR, they obtained microRNA expressions in 112 NSCLC patients, which were divided into training and testing sets. Using Cox regression and risk-score analysis, they identified a five-microRNA signature for the prediction of treatment outcome of NSCLC in the training set. This microRNA signature was validated by the testing set and an independent cohort. The method refer to a prognostic signature, with no data on a diagnostic use.
- Lebanony et al, J Clin Oncol. 2009 Apr 20;27(12):2030-7 used high-throughput microarray to measure microRNA expression levels in 122 adenocarcinoma and squamous NSCLC samples. They identified hsa-miR-205 as a highly specific marker for squamous cell lung carcinoma.
- Boeri et al Proc Natl Acad Sci USA. 2011 Mar 1;108(9):3713-8. Epub 2011 Feb 7. described a method to diagnose lung cancer in high risk individual (heavy smokers) by using circulating miRNA in plasma samples. Authors identified four different signatures of circulating micro-RNAs to be used for diagnostic and prognostic purposes. The four different miRNA signatures are: 1. A signature of risk - miRNA signature to identify subjects at risk to develop lung cancer. 2. A signature of diagnosis - miRNA signature to identify patients with lung cancer. 3. A signature of risk of aggressive disease - miRNA signature of risk to develop aggressive lung cancer. 4. A signature of presence of aggressive disease - miRNA signature to identify patients with aggressive disease. None of the above signatures anticipate the signature core method of the instant invention. Moreover Boeri et al. did not show an "authentic" case-control study, which is mandatory in order to precisely define the accuracy, sensitivity and specificity of the proposed signatures. Indeed, pools of plasma from subjects without lung cancer (i.e. the negative controls) were used instead of individual controls, thus preventing to calculate the "true" specificity of the signatures (i.e. number of control subjects predicted as negative out of the total number).
- CN101638656 discloses blood serum/blood plasmas miRNA marker related to non-small cell lung cancer (SCLC) prognosis and application thereof.
- CN101307361 discloses a method for identifying miRNA in blood serum of patient with lung cancer by Solexa technology.
- CN101608232 discloses a method for preparing novel small RNA chip for screening and identifying low-abundance small RNA expression profile and application thereof.
- US2007/050146 discloses microRNAs and use thereof.
- US2007/299030 discloses microRNA biomarkers for human breast and lung cancer.
- US2008/182237 discloses lung cancer-related nucleic acids.
- US2010/010072 discloses microRNA biomarkers for human breast and lung cancer.
- US2010/178653 discloses gene expression signature for classification of cancers.
- WO2007/081720 discloses microRNA-based methods and compositions for the diagnosis, prognosis and treatment of lung cancer. The microRNA levels are measured in lung cancer cells, not in biological fluids.
- WO2009/153775 discloses methods for distinguish between specific types of lung cancer.
- WO2009/052386 discloses microRNAs differentially expressed in lung diseases and use thereof.
- WO2009/070653 discloses some microRNA expression profiling and targeting in peripheral blood in lung cancer patients.
- WO2009/066291 discloses microRNAs expression signature for determination of tumors origin.
- WO2010/054233 discloses biomarkers in peripheral blood mononuclear cells for diagnosis or detecting lung cancers in subjects with chronic obstruction pulmonary disease or non-small cell lung cancer.
- WO2010/073248 discloses gene expression signature for classification of tissue of origin of tumor samples.
- WO2010/109017 discloses the use of miR661 as diagnostic tool for breast cancer.
- WO2010/139810 discloses methods by means of miRNA expression profiles in blood cells to confirm diagnosis of lung cancer. As a matter of fact the method has been tested on blood cells of 17 patients with NSCLC (p. 33, I. 30-33).
- WO2011/014697 discloses methods and kits by means of miRNA expression profiles, namely miR-328 and miR330-3p, to predict the development of brain metastases in NSCLC patients.
- WO2011/025919 discloses a method for characterizing lung cancer in a subject having a lung tumor by means of detecting at least one miRNA selected from a group of miRNAs in a serum sample.

None of the above signatures anticipate the signature core method of the instant invention.

### DESCRIPTION OF THE INVENTION

While early diagnosis can be successfully achieved through tomography-based population screenings in high-risk individuals, simpler methodologies are needed for effective cancer prevention programs.

Authors searched for a lung cancer diagnostic microRNAs (miRNAs) signature in biological fluids, namely serum, that could identify subjects with early stage lung tumors, in particular non-small cell lung carcinomas (NSCLCs), in a population of asymptomatic high-risk individuals. An asymptomatic high-risk individual is an individual that presents no symptoms of any pulmonary disease, while belonging to a high risk group, namely to a group having a higher probability than a reference population that a generally unfavorable outcome occurs.

Circulating microRNAs (miRNAs) were analyzed by Real Time PCR in sera of asymptomatic individuals enrolled in a large prospective early detection trial (the COSMOS study¹) started in 2004 and still ongoing, in which 5203 high-risk individuals (heavy smokers, aged over 50) were screened by annual low dose spiral computed tomography (LD-CT) to detect lung cancer. Results were validated on two independent sets of sera: 1) from asymptomatic subjects of the same trial, and 2) from an unrelated collection of sera from symptomatic patients.

Authors set up a diagnostic model of at least 5 serum miRNAs selected among a group of 34 identified miRNAs that identifies subjects with early stage lung cancer, namely NSCLCs, in a population of asymptomatic high-risk individuals with appr. 80% accuracy. The signature could also distinguish between benign and malignant lesions, is sensitive enough to capture the disease onset, diagnose or confirm a diagnosis of lung cancer in symptomatic patients and monitor the response (i.e. lung tumor status) after treatment with surgery and/or chemotherapy and/or radiotherapy in a subject with lung cancer.

It is therefore an object of the invention a method of diagnosing a lung tumor in a subject that is asymptomatic of the lung tumor, or of discriminating between benign and malignant lung nodules in a subject, or of prognosing a lung tumor in a subject, comprising the steps of:
a) detecting at least 5 miRNAs included in the following list of 34 miRNAs, in a biological fluid sample from the subject: hsa-let-7a (MIMAT0000062, SEQ ID No. 1); hsa-let-7b (MIMAT0000063, SEQ ID No. 2); hsa-let-7d (MIMAT0000065, SEQ ID No. 3); hsa-miR-103 (MIMAT0000101, SEQ ID No. 4); hsa-miR-126 (MIMAT0000445, SEQ ID No. 5); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-142-3p (MIMAT0000434, SEQ ID No. 9); hsa-miR-142-5p (MIMAT0000433, SEQ ID No. 10); hsa-miR-148a (MIMAT0000243, SEQ ID No. 11);hsa-miR-148b (MIMAT0000759, SEQ ID No. 12); hsa-miR-17 (MIMAT0000070, SEQ ID No. 13); hsa-miR-191 (MIMAT0000440, SEQ ID No. 14); hsa-miR-22 (MIMAT0000077, SEQ ID No. 15); hsa-miR-223 (MIMAT0000280, SEQ ID No. 16); hsa-miR-26a (MIMAT0000082, SEQ ID No. 17); hsa-miR-26b (MIMAT0000083, SEQ ID No. 18); hsa-miR-28-5p (MIMAT0000085, SEQ ID No. 19); hsa-miR-29a (MIMAT0000086, SEQ ID No. 20); hsa-miR-30b (MIMAT0000420, SEQ ID No. 21); hsa-miR-30c (MIMAT0000244, SEQ ID No. 22); hsa-miR-32 (MIMAT0000090, SEQ ID No. 23); hsa-miR-328 (MIMAT0000752, SEQ ID No. 24); hsa-miR-331-3p (MIMAT0000760, SEQ ID No. 25); hsa-miR-342-3p (MIMAT0000753, SEQ ID No. 26); hsa-miR-374a (MIMAT0000727, SEQ ID No. 27); hsa-miR-376a (MIMAT0000729, SEQ ID No. 28); hsa-miR-432-staR (MIMAT0002815, SEQ ID No. 29); hsa-miR-484 (MIMAT0002174, SEQ ID No. 30); hsa-miR-486-5p (MIMAT0002177, SEQ ID No. 31); hsa-miR-566 (MIMAT0003230, SEQ ID No. 32); hsa-miR-92a (MIMAT0000092, SEQ ID No. 33); hsa-miR-98 (MIMAT0000096, SEQ ID No. 34); and
b) comparing the amounts of said miRNAs to proper controls, wherein said comparison allows for diagnosing or prognosing a lung tumor, or for discriminating between benign and malignant lung nodules.

In a preferred embodiment said at least 5 miRNAs are: hsa-let-7a (MIMAT0000062, SEQ ID No. 1); hsa-miR-133b (MIMAT0000770, SEQ ID No. 6); hsa-miR-139-5p (MIMAT0000250, SEQ ID No. 7); hsa-miR-342-3p (MIMAT0000753, SEQ ID No. 26); hsa-miR-486-5p (MIMAT0002177, SEQ ID No. 31).

In a particular embodiment of the method of the invention the detecting step a) comprises the detecting of at least 10 miRNAs included in the list of above 34 miRNAs.

In a preferred embodiment said at least 10 miRNAs are: hsa-let-7a (MIMAT0000062, SEQ ID No. 1); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-148b (MIMAT0000759, SEQ ID No. 12); hsa-miR-26b (MIMAT0000083, SEQ ID No. 18); hsa-miR-32 (MIMAT0000090, SEQ ID No. 23); hsa-miR-342-3p (MIMAT0000753, SEQ ID No. 26); hsa-miR-432-staR (MIMAT0002815, SEQ ID No. 29); hsa-miR-486-5p (MIMAT0002177, SEQ ID No. 31).

In a particular embodiment of the method of the invention the detecting step a) comprises the detecting of at least 15 miRNAs included in the list of the above 34 miRNAs.

In a preferred embodiment said at least 15 miRNAs are: hsa-let-7a (MIMAT0000062, SEQ ID No. 1); hsa-let-7b (MIMAT0000063, SEQ ID No. 2); hsa-let-7d (MIMAT0000065, SEQ ID No. 3); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-148b (MIMAT0000759, SEQ ID No. 12); hsa-miR-17 (MIMAT0000070, SEQ ID No. 13); hsa-miR-26b (MIMAT0000083, SEQ ID No. 18); hsa-miR-29a (MIMAT0000086, SEQ ID No. 20); hsa-miR-32 (MIMAT0000090, SEQ ID No. 23); hsa-miR-342-3p (MIMAT0000753, SEQ ID No. 26); hsa-miR-432-staR (MIMAT0002815, SEQ ID No. 29); hsa-miR-486-5p (MIMAT0002177, SEQ ID No. 31); hsa-miR-92a (MIMAT0000092, SEQ ID No. 33).

In a particular embodiment of the method of the invention the detecting step a) comprises the detecting of at least 20 miRNAs included in the list of the above 34 miRNAs.

In a preferred embodiment said at least 20 miRNAs are: hsa-let-7a (MIMAT0000062, SEQ ID No. 1); hsa-let-7b (MIMAT0000063, SEQ ID No. 2); hsa-let-7d (MIMAT0000065, SEQ ID No. 3); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-148b (MIMAT0000759, SEQ ID No. 12); hsa-miR-22 (MIMAT0000077, SEQ ID No. 15); hsa-miR-26a (MIMAT0000082, SEQ ID No. 17); hsa-miR-26b (MIMAT0000083, SEQ ID No. 18); hsa-miR-28-5p (MIMAT0000085, SEQ ID No. 19); hsa-miR-29a (MIMAT0000086, SEQ ID No. 20); hsa-miR-30b (MIMAT0000420, SEQ ID No. 21); hsa-miR-32 (MIMAT0000090, SEQ ID No. 23); hsa-miR-342-3p (MIMAT0000753, SEQ ID No. 26); hsa-miR-432-staR (MIMAT0002815, SEQ ID No. 29); hsa-miR-484 (MIMAT0002174, SEQ ID No. 30); hsa-miR-486-5p (MIMAT0002177, SEQ ID No. 31); hsa-miR-92a (MIMAT0000092, SEQ ID No. 33); hsa-miR-98 (MIMAT0000096, SEQ ID No. 34).

In a particular embodiment of the method of the invention the detecting step a) comprises the detecting of at least 25 miRNAs included in the list of the above 34 miRNAs.

In a preferred embodiment said at least 25 miRNAs are: hsa-let-7a (MIMAT0000062, SEQ ID No. 1); hsa-let-7b (MIMAT0000063, SEQ ID No. 2); hsa-let-7d (MIMAT0000065, SEQ ID No. 3); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-148b (MIMAT0000759, SEQ ID No. 12); hsa-miR-17 (MIMAT0000070, SEQ ID No. 13); hsa-miR-22 (MIMAT0000077, SEQ ID No. 15); hsa-miR-223 (MIMAT0000280, SEQ ID No. 16); hsa-miR-26a (MIMAT0000082, SEQ ID No. 17); hsa-miR-26b (MIMAT0000083, SEQ ID No. 18); hsa-miR-28-5p (MIMAT0000085, SEQ ID No. 19); hsa-miR-29a (MIMAT0000086, SEQ ID No. 20); hsa-miR-30b (MIMAT0000420, SEQ ID No. 21); hsa-miR-32 (MIMAT0000090, SEQ ID No. 23); hsa-miR-328 (MIMAT0000752, SEQ ID No. 24); hsa-miR-342-3p (MIMAT0000753, SEQ ID No. 26); hsa-miR-374a (MIMAT0000727, SEQ ID No. 27); hsa-miR-432-staR (MIMAT0002815, SEQ ID No. 29); hsa-miR-484 (MIMAT0002174, SEQ ID No. 30); hsa-miR-486-5p (MIMAT0002177, SEQ ID No. 31); hsa-miR-566 (MIMAT0003230, SEQ ID No. 32); hsa-miR-92a (MIMAT0000092, SEQ ID No. 33); hsa-miR-98 (MIMAT0000096, SEQ ID No. 34).

In a particular embodiment of the method of the invention the detecting step a) comprises the detecting of at least 30 miRNAs included in the list of the above 34 miRNAs.

In a preferred embodiment said at least 30 miRNAs are: hsa-let-7a (MIMAT0000062, SEQ ID No. 1); hsa-let-7b (MIMAT0000063, SEQ ID No. 2); hsa-let-7d (MIMAT0000065, SEQ ID No. 3); hsa-miR-103 (MIMAT0000101, SEQ ID No. 4); hsa-miR-126 (MIMAT0000445, SEQ ID No. 5); hsa-miR-133b (MIMAT0000770, SEQ ID No. 6); hsa-miR-139-5p (MIMAT0000250, SEQ ID No. 7); hsa-miR-140-5p (MIMAT0000431, SEQ ID No. 8); hsa-miR-148b (MIMAT0000759, SEQ ID No. 12); hsa-miR-17 (MIMAT0000070, SEQ ID No. 13); hsa-miR-191 (MIMAT0000440, SEQ ID No. 14); hsa-miR-22 (MIMAT0000077, SEQ ID No. 15); hsa-miR-223 (MIMAT0000280, SEQ ID No. 16); hsa-miR-26a (MIMAT0000082, SEQ ID No. 17); hsa-miR-26b (MIMAT0000083, SEQ ID No. 18); hsa-miR-28-5p (MIMAT0000085, SEQ ID No. 19); hsa-miR-29a (MIMAT0000086, SEQ ID No. 20); hsa-miR-30b (MIMAT0000420, SEQ ID No. 21); hsa-miR-30c (MIMAT0000244, SEQ ID No. 22); hsa-miR-32 (MIMAT0000090, SEQ ID No. 23); hsa-miR-328 (MIMAT0000752, SEQ ID No. 24); hsa-miR-342-3p (MIMAT0000753, SEQ ID No. 26); hsa-miR-374a (MIMAT0000727, SEQ ID No. 27); hsa-miR-376a (MIMAT0000729, SEQ ID No. 28); hsa-miR-432-staR (MIMAT0002815, SEQ ID No. 29); hsa-miR-484 (MIMAT0002174, SEQ ID No. 30); hsa-miR-486-5p (MIMAT0002177, SEQ ID No. 31); hsa-miR-566 (MIMAT0003230, SEQ ID No. 32); hsa-miR-92a (MIMAT0000092, SEQ ID No. 33); hsa-miR-98 (MIMAT0000096, SEQ ID No. 34).

In a particular embodiment of the method of the invention the detecting step a) comprises the detecting of all miRNAs included in the list of the above 34 miRNAs.

In a preferred embodiment the biological fluid sample is comprised in the group of: blood, serum, plasma, urine, saliva, mucus, tears, amniotic fluid, breast milk, sputum, cerebrospinal fluid, peritoneal fluid, pleural fluid and seminal fluid, or fractions thereof. After taking the biological fluid sample from the subject, the same may also be stored, frozen, lyophilized, fractionated, etc.

In a preferred embodiment the lung tumor is a non-small cell lung carcinoma (NSCLC).

In a preferred embodiment wherein the detection of said miRNAs is performed by means of hybridization with primers and/or probes, each one specific and selective for the sequence of one of miRNAs. Any other suitable method of hybridization and detection of nucleic acid are within the scope of the invention, as microarray or sequencing.

In a preferred embodiment of the invention proper controls comprise same miRNAs from healthy subjects, namely subject with no lung tumors.

In a preferred embodiment the method of the invention comprises the step of normalizing the amounts of said miRNAs with at least one unrelated miRNA present in the same biological fluid sample, and/or with at least one unrelated miRNA exogenously spiked in the sample.

Synthetic microRNA spikes may be used to measure the *recovery* of total microRNAs during the method protocol. Spike microRNAs are advantageously selected from non-human microRNAs (i.e. microRNAs without any homologous in human), also in function of the technical platform.

In a preferred embodiment the at least one unrelated miRNA belongs to the group of miR-197, SEQ ID No. 35; miR-19b, SEQ ID No. 36; miR-24, SEQ ID No. 37; miR-146a, SEQ ID No. 38; miR-15b, SEQ ID No. 39; miR-19a, SEQ ID No. 40. More preferably said unrelated miRNAs are miR-197, SEQ ID No. 35; miR-19b, SEQ ID No. 36; miR-24, SEQ ID No. 37; miR-146a, SEQ ID No. 38; miR-15b, SEQ ID No. 39; miR-19a, SEQ ID No. 40.

In a preferred embodiment the method of the invention comprises the step of normalizing the amounts of core 5-34 miRNAs with the median of the amount of unrelated miRNAs present in or added to the same biological fluid sample. Another aspect of the invention refers to a method of monitoring a lung tumor status after treatment with surgery and/or chemotherapy and/or radiotherapy in a subject with said lung tumor, comprising the step of following the modulation of core 5-34 miRNAs.

Another aspect of the invention refers to a kit to perform the method as above disclosed comprising specific primers and/or probes for each of miRNAs to be detected.

Another aspect of the invention refers a microarray or a polymerase chain reaction (PCR) plate to perform the method as above disclosed comprising specific probes for each of miRNAs to be detected.

### ADVANTAGES OF THE INVENTION

The test can identify subjects with early stage NSCLC in asymptomatic high-risk individuals. The test displays several characteristics that are desirable in a clinical setting:
1) it can be performed on low amounts of biological fluids (i.e. serum, 0.1-1.0 ml or more) without any need for a pre-amplification step,
2) it is considerably cheaper, easier and more immediately implementable (particularly from the point of view of patient accrual and compliance) than current screening procedures,
3) it assigned disease probability accurately in two independent testing cohorts of subjects representing either asymptomatic individuals (the COSMOS testing set A) or symptomatic patients (the independent testing set B),
4) it could distinguish between benign and malignant lesions, and capture the onset of the malignant disease.

When compared to LD-CT-based screening protocols, the most powerful tool for early diagnosis available, the test of the invention showed an accuracy of 80%, and was remarkably stable between training and testing sets.

The test finds its main application in the clinic as a "first line screening test" for high-risk individuals, to identify those who should undergo further testing, including by LD-CT. Such a test might prove very useful for high-risk population screening with a cheap and minimally invasive procedure with no needs for specific accrual. Furthermore, the test also finds an application in the clinic as a "second line screening test" to diagnose cancer in case of a LD-CT detected (or by other diagnostic tools) lung nodule, and/or to monitor disease regression after therapeutic intervention.

The expert shall realize that a larger study that systematically and prospectively compares the results of LD-CT and the serum test of the invention may adjust the cut-off score or establish a cut-off range of the multivariate risk predictor. Such variations are within the scope of the invention provided that they would not change the composition of the core "at least 5 miRNAs among the 34-miRNA group" model, given its remarkable stability.

### DETAILED DESCRIPTION OF THE INVENTION

The invention is now described in specific non limiting examples.

### FIGURE LEGENDS

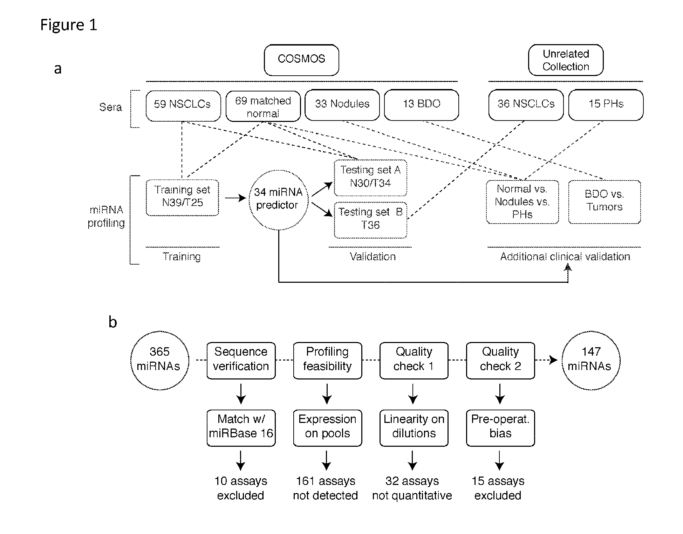

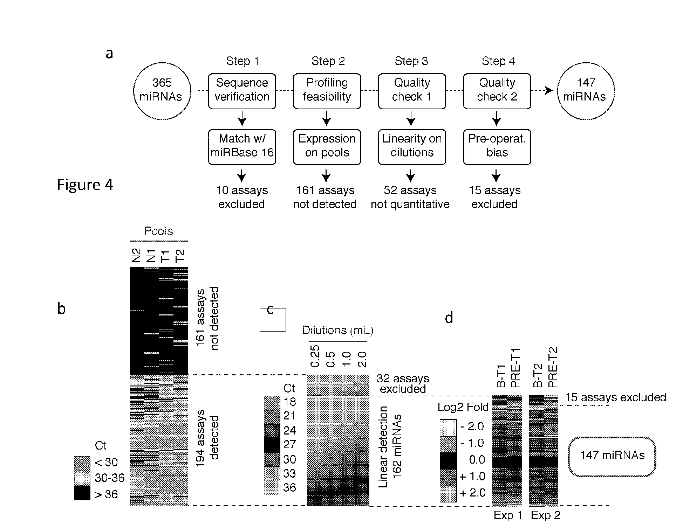

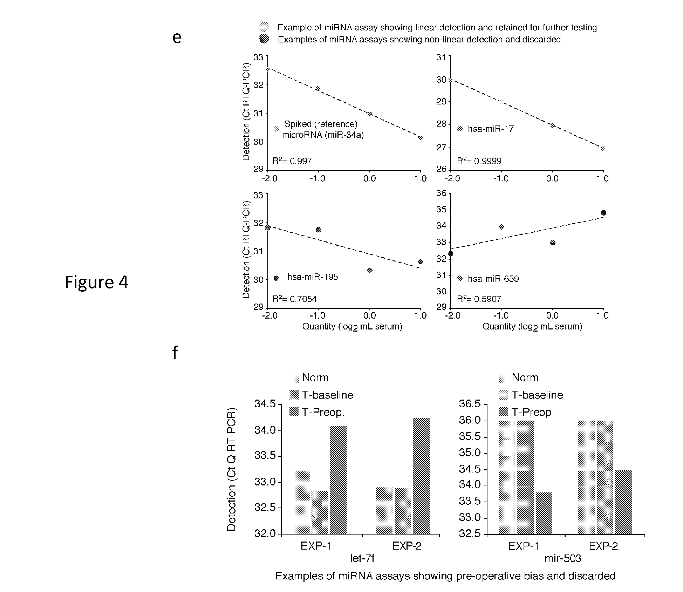

**Figure 1****. Study design. a.** Sera were obtained from two independent collections: i) form the COSMOS study [59 non-small cell lung carcinomas (NSCLCs), 69 Normal, 33 Nodules, 13 sera before disease onset (BDO)], and ii) from an unrelated serum collection [36 NSCLCs (from symptomatic patients), 15 pulmonary hamartomas (PHs)]. COSMOS NSCLC and Normal sera were divided into a training set and a testing set A (N, normal; T, tumor). The testing set B consisted of NSCLC sera from the unrelated collection. Additional sets were used for other clinical validations, as shown in the Figure and explained in the main text. **b.** A total of 365 miRNA assays were employed in the screening. A series of tests was implemented to exclude those miRNAs that did not meet a number of stringent criteria for inclusion in the final analysis (see Figure 4 for details). This led to the selection of 147 miRNAs that were used for all analyses presented in the study.

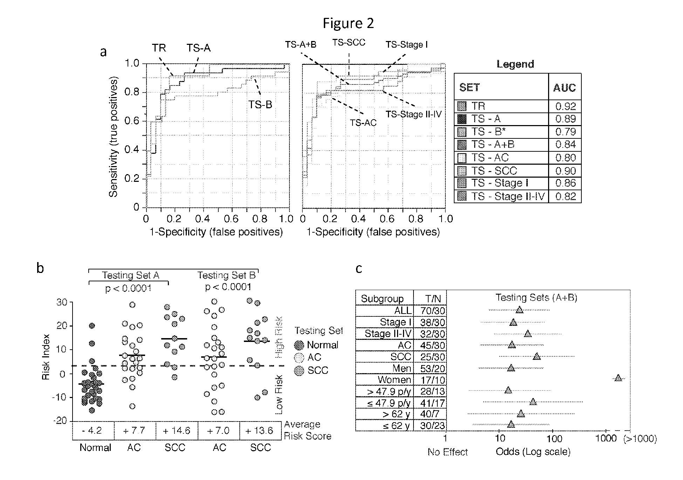

**Figure 2****. The 34-miRNA diagnostic model. a.** Receiver operating characteristic (ROC) curves of the 34-miRNA diagnostic model (curves are presented in two separate panels solely for reasons of clarity). Color codes and dashed lines are as per the "legend". TR, training set; TS-A, testing set A; TS-B, testing set B; TS-AC, testing set A+B considering only AC (adenocarcinoma); TS-SCC (squamous cell carcinoma), testing set A+B considering only SCCs; TS-Stage I, testing set A+B considering only stage I tumors; TS-Stage II-IV, testing set A+B considering all other tumor stages. *, since in the testing set B "normal" sera were not present, the normal values from the testing set A were used instead. AUC, area under curve. **b.** Risk of cancer in NSCLC patients (separately for ACs and SCCs) in the testing sets, based on the 34-miRNA risk model. The dashed line indicates the DLDA decision cut-off (3.235 risk). Average risk scores and P-values (ANOVA) are also shown. **c.** Forest plot showing the 34-miRNA model prediction strength in the testing sets (ALL, 30 Normal and 70 tumors) stratified by available clinical-pathological parameters. Triangles represent the odds ratios; lines represent the relative 95% confidence intervals (Nominal logistic regression). Age (years) and packs/year (p/y) cut-offs were defined by the relative averages in the 100 subjects. P-values were < 0.005 in all analyses (two-tails Fisher's exact test). Stage I, lung tumor stage I; Stage II-IV, lung tumor stage II, III or IV.

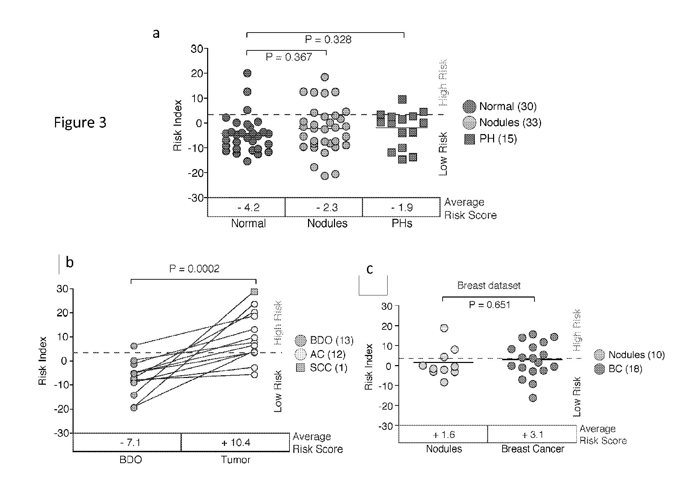

**Figure 3****. Performance of the 34-miRNA diagnostic model under various conditions of clinical interest. a.** Risk index in subjects with benign nodules (nodules), pulmonary hamartomas (PHs), or normal individuals (normal). The dashed line shows the DLDA decision cut-off (3.235). Average risk scores and P-values (Welch's t-test) are also shown. **b.** Risk index in subjects before disease onset (BDO) and after the onset of disease (Tumor, AC and SCC are shown by circles and squares respectively). Average risk scores and P-values (one-tailed paired t-test) are also shown. **c.** Risk index in patients with breast cancer (BC, Breast Cancer) or benign breast nodules (nodules). Average risk scores and P-values (Welch's t-test) are also shown.
**Figure 4****. Serum miRNA tests. Methodology.** For serum isolation, blood was kept at RT for 30-60 minutes to clot, then spun at 3000 rpm (1000-1300 g) for 10 minutes. The serum was removed and dispensed in 1 ml aliquots into 2 ml cryotubes. Specimens were stored at -80 C. Serum RNA (from 1 ml of sera, 0.5 ml for sera of the testing set B) was extracted with Trizol-LS (Invitrogen) combined with mirVana miRNA Isolation Kit (Ambion). Briefly, Trizol-LS was added to serum in volumetric ratios of 3:1 (3 ml Trizol for 1 mL serum) according to manufacturer's instructions. After the chloroform-addition step and phase separation, the aqueous phase was mixed with 1.25 volumes of absolute ethanol. The resulting solution was then loaded onto the cartridge provided in the mirVana miRNA Isolation Kit (Ambion), and total RNA was eluted in 50 µL of water and stored at -80°C. MiRNA levels were analyzed with the TaqMan® Low Density Array microRNA Signature Panel (v1.0), containing 365 human miRNA assays (Applied Biosystems). Briefly, RNA was reverse transcribed using the TaqMan MiRNA Riverse Transcription Kit, and the TaqMan Multiplex RT assays, Human pool set. Each of the eight multiplex RT- reactions was performed using 4.75 µL of extracted RNA (50 µL from 1 mL serum) in a final volume of 20 µL. Each multiplex RT reaction was then diluted to 50 µL with water and combined with 50 µL of TaqMan 2X Universal PCR master Mix, No AmpErase UNG. The final solution (100 µL) was loaded into the array, and RTQ-PCR was carried out on an Applied Biosystems 7900HT thermocycler using the manufacturer's recommended cycling conditions. **a. Selection of miRNA assays.** A total of 365 miRNA assays were initially utilized in the screening. A series of tests was implemented to exclude those miRNAs that did not meet a number of stringent criteria for inclusion in the final analysis. In step 1 (sequence verification), 10 assays were excluded because they did not exactly match the mature miRNA sequence in the latest miRNA database (miRbase 16.0). In step 2 (profiling feasibility, see also panel B), 161 assays were excluded because they could not be detected reproducibly in sera. In step 3 (quality check 1, see also panels C, E), 32 assays were excluded because they did not show linear behavior in the detection. In step 4 (quality check 2, see also panels D, F), 15 assays were excluded, because they were possibly affected by preoperative treatments. At the end of these calibrations, 147 miRNA assays were considered suitable for our purposes and subjected to the analyses described in the main text. **b. Details of Step 2.** We selected only those miRNAs that could be detected reproducibly in sera. For this purpose, we prepared 4 pooled serum samples (2 from normal sera - N1 and N2 pools -, and 2 from tumor sera - T1 and T2 pools -; all sera were from the training set), each containing equal amounts of sera from 5 donors. Total RNA was extracted from each pool and used for miRNA analysis by RTQ-PCR. The data matrix shows the detection of miRNA assays (based on Ct value cut-offs) in the four pools (columns, pools; rows, miRNAs). Assays with a Ct value > 36, were considered not detected, (black), assays with a Ct value < 36 were considered detected (dark grey Ct < 30, light grey for Ct 30-36). Only assays detected in at least 2 of 4 pools (194 assays) were retained; the others (161 assays) were excluded from further analyses. **c. Details of Step 3.** Next, we excluded those miRNA assays that did not display linear behavior of detection. A pool of sera from 20 healthy individuals was used for this purpose. As an internal control, we added a known amount of a synthetic miRNA (miR-34a, which was never detected in serum) to the pool before dilution (10⁷ copies in 100 µL of total RNA). The known amount of miR-34a was used to calibrate the measurement of miRNA copies in the serum and evaluate the sensitivity of the platform. The pool of sera was then subjected to linear two-fold dilutions, and used for miRNA analysis by RTQ-PCR. Ct values for each miRNA were plotted on a log scale (log quantity against expression Ct) and values were fitted by linear regression. Assays which showed a coefficient of determination (R2) of < 0.85 were discarded, and 162 assays were retained for further analyses. The data matrix shows the expression profile of miRNA assays in the four dilutions. Examples of individual miRNA assays are shown in panel E. **d. Details of Step 4.** The vast majority of sera from the COSMOS study were collected prior to the LD-CT scan. In some cases of NSCLC patients from the COSMOS study, however, sera collected at the baseline were not available; therefore we used sera taken before surgery (which normally took place a few days or weeks after the LD-CT scan). In addition, the sera of the testing cohort B were all taken pre-surgery. In all cases of pre-surgery sera, subjects underwent diagnostic procedures before serum collection. Thus, it was important to assess whether a pre-operative bias would affect the levels of some miRNAs; in other words, whether some miRNAs could fluctuate in serum as a result of preoperative diagnostic procedures. To determine this, we prepared four pools of sera (5 subjects/pool) by grouping together matched tumor samples taken from the same LD-CT-detected NSCLC patients at two close time points (<60 days): at the baseline (prior to LD-CT, pool b-T1 and b-T2) and before surgery (pre-operative, pool pre-T1 and pre-T2). Two other pools were generated by pooling sera from healthy individuals, always from COSMOS sera, (pool N1 and N2) and used as a reference to calculate microRNA regulation in each condition. Total RNA was extracted from each pool and used for microRNA analysis by RTQ-PCR. The data matrix shows the regulation of each microRNA at the baseline (pool b-T over pool N) and at preoperative stage (pool pre-T over pool N) in two different experiments (EXP1, N1 vs. b-T1 vs. pre-T1; EXP2, N2 vs. b-T2 vs. pre-T2). A total of 15 miRNA assays were reproducibly regulated only at the preoperative stage and were, therefore, excluded from further analyses. Examples are shown in panel F. e. examples of miRNA selection at step 3. f. Examples of miRNA selection at step 4.

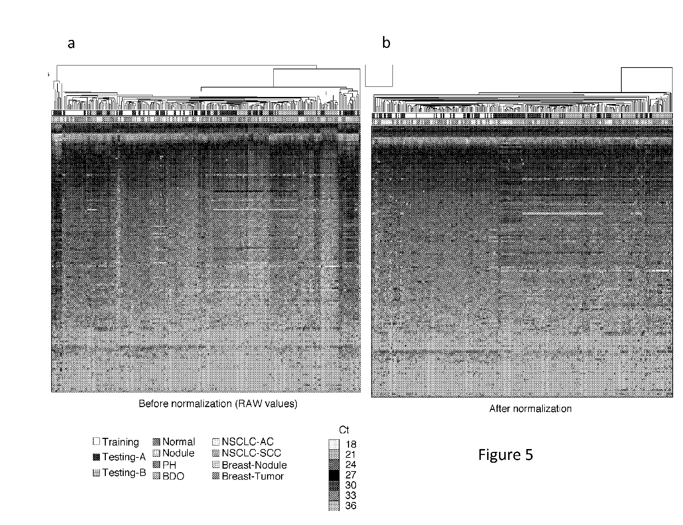

**Figure 5****. Serum miRNA dataset.** Expression profile data matrix of the 147 miRNAs in all the analyzed sera. A total of 253 sera (whose origin is indicated in the legend) were profiled. Data matrices show the Ct values of each microRNA before (**a**) and after (**b**) normalization. Grey bars indicate the class of each subject. Columns, subjects; rows, miRNAs.

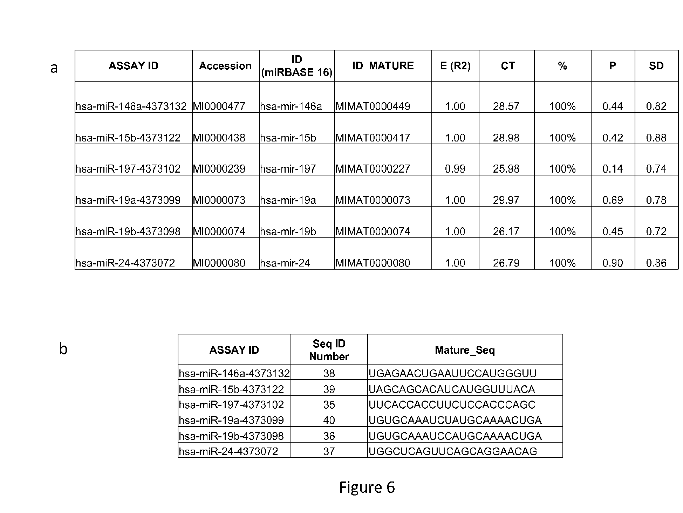

**Figure 6****. a** The serum housekeeping (HK) miRNAs selected in the training set are shown (with indication of the assay ID, accession number, ID in miRbase16 release database, and accession number ID of mature miRNA). E, efficiency of the RTQ assay, expressed as coefficient of determination (R2), and determined as illustrated in Figure 4. Also shown are the parameters used for the selection of the HK in the training cohort: Ct (median) of expression (which had to be less than 30 for inclusion); percentage (%) of sample with RTQ detection (which had to be 100% for inclusion), pvalue (P) by Welch T-test in the training set (which had to be > 0.10 for inclusion), and Ct standard deviation (SD) in the training set (which had to be < 0.90 for inclusion). **b** The mature sequences of same serum housekeeping (HK) miRNAs of panel **a** are shown with indication of the assay ID and of the Sequence Listing numbering.

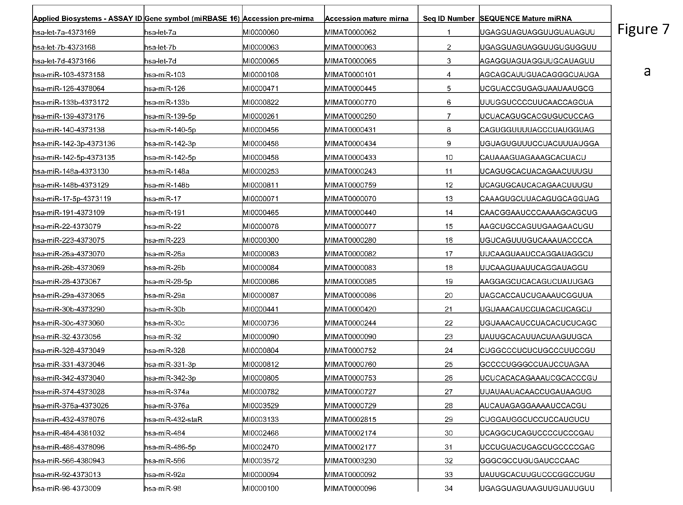

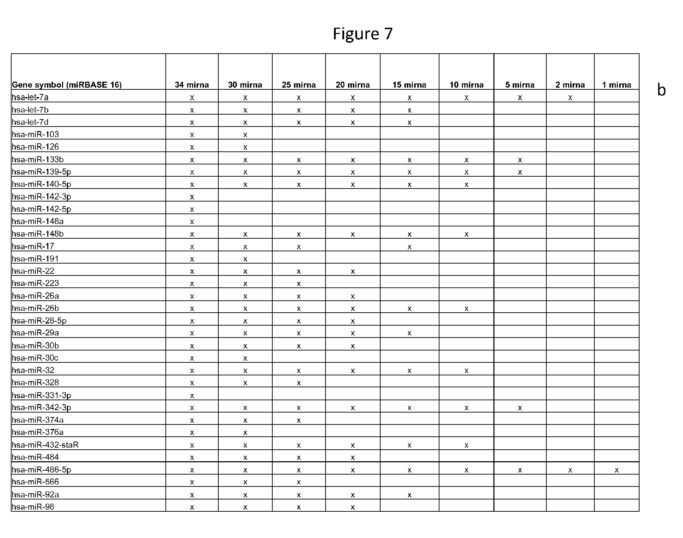

**Figure 7****.** The different diagnostic models based on combinations of selected miRNAs are shown. **a** The 34 core miRNAs are shown with relative Applied Biosystems assay ID, gene symbol (miRBASE release 16 database), accession numbers of pre-miRNA and mature miRNA, Sequence Listing numbering and mature miRNA sequences. **b** The gene symbol (miRBASE release 16 database) of the same 34 miRNAs of panel **a** is repeated with selection of different core miRNAs models (labeled by a cross).

### METHODS

### Subject selection criteria

Patients with NSCLC and healthy individuals were selected from a consecutive series of 5203 subjects enrolled in the COSMOS trial¹. All were smokers or former-smokers with a smoking exposure of more than 20 pack-years, aged over 50 (Table 1). Tumor stage at the time of diagnosis was determined according to guidelines of the American Joint Committee on Cancer (http://www.cancerstaging.org/). Informed consent was obtained from all subjects.

**Table 1. Clinical characteristics of subjects of the training and testing sets**

| **Categories** | **Training Set (N = 64)** | **Testing Set A (N= 64)** | **Testing Set B (N = 36)** |
|---|---|---|---|
| Tumor | 25 | 34 | 36 |
| Normal | 39 | 30 | 0 |
| Male | 45 | 43 | 30 |
| Female | 19 | 21 | 6 |
| Non-smokers | 0 | 0 | 8 |
| Age (years) | 58±6 | 60±6 | 66±8 |
| Pack-years | 54±18 | 49±17 | 45±37 |

| **Tumor Subtype** | | | |
|---|---|---|---|
| AC | 25 | 22 | 23 |
| SCC | 0 | 12 | 13 |

| **Tumor Stage** | | | |
|---|---|---|---|
| IA | 19 | 18 | 7 |
| IB | 2 | 4 | 9 |
| II-IV | 4 | 12 | 20 |

| | | | |
|---|---|---|---|
| **Table 1.** The clinical and pathological characteristics are reported for the training and testing sets of subjects (N, number of subjects). AC, adenocarcinoma; SCC, squamous cell carcinoma. The average and relative standard deviation (± SD) is indicated for Age (years) and smoking status (Pack-years). Stage IA and IB, lung tumor stage IA and IB; Stage II-IV, lung tumor stage II, III or IV. Other cohorts of subjects, employed in the study, are described in the main text. | | | |

### miRNA profiling and data normalization

Serum preparation and RNA extraction are described in Figure 4. RNAs were isolated from 1 ml of serum (0.5 ml for sera of the testing set B). MiRNA levels were analyzed with the TaqMan® Low Density Array microRNA Signature Panel (v1.0) (Applied Biosystems), as described in Figure 4. Data were analyzed with SDS Relative Quantification Software version 2.2.2 (Applied Biosystems). Ct values were exported into Excel software (Microsoft) for data analysis.

According to the analyses described in Figure 4, only 147 assays were suitable for the purpose of our study, thus only these assays were considered for data normalization and further analyses. In the training set, we selected six miRNAs behaving as "housekeeping (HK) serum-miRNAs" (miR-197, SEQ ID No. 35; miR-19b, SEQ ID No. 36; miR-24, SEQ ID No. 37; miR-146a, SEQ ID No. 38; miR-15b, SEQ ID No. 39; miR-19a, SEQ ID No. 40; Fig. 6) with the following criteria: i) they passed all the quality tests described in Figure 4, in particular, they were optimal quantitative variables (R² = 0.99), ii) they were expressed at high levels and in all samples (median Ct<30; 100% of detection); iii) they were not statistically different among the analyzed classes (Welch's t-test, p > 0.1); iv) they were the least variable miRNAs among all samples (St Dev < 0.9 Ct). Raw data were therefore normalized by the geometric mean of the "housekeeping (HK) serum-miRNAs".

Only values below a minimal threshold (Ct < 36) were normalized in order to avoid artifactual regulation due to sample normalization. Values for the testing sets A and B were normalized using the same HK serum-miRNAs. Expression values for all serum samples used in the study, before and after normalization, are shown in Figure 5.

The performance of the 34-miRNA model using this method of normalization is shown in Table 2.

To further control for the robustness of our findings, authors repeated the evaluation of the performance of the 34-miRNA model using a different method for normalization, the "median" method in which data were normalized based on the median Ct value of all the 147 detected miRNAs (Table 3).

### Use of spikes in serum miRNA extraction and quantification

Authors envision the use of synthetic microRNA spikes to measure the *recovery* of total microRNAs during the method protocol. This is relevant since the extraction of microRNAs from serum is complicated by a number of factors, (the main one being lipid/protein content of each sample). There are two distinct steps that should be monitored, requiring preferably the addition of two different spikes:
1- Extraction efficiency: a first spike is added to serum immediately after the denaturing step (otherwise the spike would be degraded by RNAses), to measure the efficiency in microRNA isolation step
2- Reverse trascription efficiency: a second spike is added to extracted microRNAs (i.e. the final product of step 1, consisting of purified microRNAs in water), to measure the efficiency of the cDNA synthesis step.

Spike 1 and spike 2 are advantageously selected from non-human microRNAs (i.e. microRNAs without any homologous in human), and also selected in function of the technical platform used for miRNA detection.

### Clustering and class prediction analyses

Hierarchical clustering analysis was performed using Cluster 3.0 for Mac OSX (http://bonsai.hgc.jp/-mdehoon/software/cluster/software.htm). Expression data were clustered using uncentered correlation and average linkage. Tree pictures were generated using Java TreeView software (http://jtreeview.sourceforge.net).

For class prediction, log₂ median centered expression data were analyzed using BRB-ArrayTools Version: 3.8.0-Beta_1 Release (April 2009) (http://linus.nci.nih.govBRB-ArrayTools.html). miRNAs significantly different between case and control classes in the training set, at 0.05 significance level (parametric t-test, random variance model), were used for class prediction. The misclassification rate of the classifier was computed by DLDA (diagonal linear discriminant analysis) and K-fold (K=5) cross-validation method repeated 100 times. Statistical significance of the DLDA classifier was assessed by 1000 random permutations of the class labels. The classification of subjects in the two testing sets was performed blinded, using the following prediction rule from the Diagonal linear Discriminant predictor: a sample is classified high risk/with tumor if the inner sum of the weights (*wᵢ*) and expression (*xᵢ*) of the 34 miRNAs is greater than the threshold (determined in the training set); that is, ∑ *iwᵢ xᵢ* > 3.235. Statistical significance of the differences of the average risk index in the various sets of subjects was calculated using ANOVA (in the case of more than two groups) or Welch's t-test using Prism (GraphPad Software, Inc.). Statistical significance of the differences of the average risk index between BDO and relative matched tumor sera was calculated using the one-tail paired t-test (GraphPad Software, Inc.). Sensitivity analyses and Forest Plots were prepared using the statistical software JMP IN (SAS), and P values calculated with the Fisher's exact test.

For selection of different miRNA combinations, authors imposed a fixed number of miRNAs (34, 30, 25, 20, 15, 10, 5, 2, 1) that were used for class prediction. The misclassification rate of the classifier was computed by DLDA (diagonal linear discriminant analysis) and K-fold (K=5) cross-validation method repeated 100 times. Statistical significance of the DLDA classifier was assessed by 1000 random permutations of the class labels.

### RESULTS

### Serum miRNAs in asymptomatic NSCLC subjects and healthy smokers

A large study (the COSMOS study¹) in which 5203 high-risk individuals were screened by annual LD-CT to detect lung cancer (Fig. 1A) was used. Ninety-three NSCLCs were diagnosed in the first two years of screening (55 at baseline and 38 at annual screening). Sera - collected before surgery - were available for 59 of these subjects (Fig. 1A). At the time of serum collection, all subjects were asymptomatic, and the detection of cancer was solely due to their enrolment in the observational project. We selected control sera (matched for confounding factors, such as gender, age and smoking exposure) from 69 individuals enrolled in the same study, in whom no cancer was found by LD-CT during the entire study (healthy smokers, henceforth "normal") (Fig. 1A).

Sera from the COSMOS study were divided into two sets: a training set (39 normal subjects and 25 with adenocarcinomas - AC) and a testing set (testing set A, 30 normal subjects, 22 AC, and 12 squamous cell carcinomas - SCC) (Fig. 1A, and Table 1). To circumvent possible "study biases", we also analyzed an independent testing set (testing set B, 23 AC and 13 SCC, Fig. 1A and Table 1), consisting of pre-operative sera collected during different years at the European Institute of Oncology, as part of the clinical activity, from subjects with symptomatic NSCLC.

A total of 365 miRNA assays were employed in the study. A series of calibration tests (schematized in Fig. 1B and described in Figure 4) were performed to select a pool of 147 miRNAs that were informative (see Methods and Figure 4 for details) for the purpose of our study. The levels of these 147 miRNAs were evaluated in RNAs extracted from all the training/testing sera. After miRNA profiling and normalization (see Methods and Figure 5), we used the training set to derive a 34-miRNA signature capable of separating tumor from normal sera (Methods, Fig. 7).

For selection of different miRNA combinations, authors imposed a fixed number of miRNAs (34, 30, 25, 20, 15, 10, 5, 2, 1) that were used for class prediction (Fig. 7). The misclassification rate of the classifier was computed as explained in Materials and Methods.

### An at least 5 from 34-miRNA model for NSCLC diagnosis

Authors developed a multivariate risk predictor, using the weighted linear combination of the 34 miRNA expression values (see Methods), to assign each subject to a high (i.e. with lung cancer) or low risk category (with a cut-off score set at 3.235 during the training of the classifier which was performed on the training set). In the training set, the risk algorithm displayed an accuracy of 78% and an area under the curve (AUC) of 0.92 (Table 2 Fig. 2A). The predictor was remarkably stable when applied to the testing sets, displaying an accuracy of 80% in both testing sets (A and B) and AUCs of 0.89 and 0.79, respectively (Table 2, Fig. 2A). Importantly, the algorithm, which was derived on a training set containing only ACs, performed well in both SCCs and ACs in the testing sets (Table 2, Fig. 2A-B). In addition, in the testing sets, in which a number of more advanced cancers were present with respect to the training set, the predictor performed comparably well for cancers of all stages I-IV (Table 2,Fig. 2A). Finally, a sensitivity analysis showed that the 34-miRNA signature remained a strong predictor of risk independently of the subgroups of subjects considered (Fig. 2C).

**Table 2 Performance of the predictive model in various sets**

| **SET** | **ACC %** | **SEN %** | **SPE %** | **AUC** |
|---|---|---|---|---|
| **Training set** (39 N, 25 AC) | 78^{§} | 69^{§} | 84^{§} | 0.92 |
| **Testing set A** (30 N, 22AC, 12 SCC) | 80 | 71 | 90 | 0.89 |
| **Testing set B** (30 N*, 23 AC, 13 SCC) | 80 | 72 | 90 | 0.79 |
| **Testing set A+B** (30 N, 45 AC, 25 SCC) | 77 | 71 | 90 | 0.84 |
| **Testing set A+B - AC only** (30 N, 45 AC) | 75 | 64 | 90 | 0.80 |
| **Testing set A+B - SCC only** (30 N, 25 SCC) | 87 | 84 | 90 | 0.90 |
| **Testing set A+B - Stage I only** (30 N, 38 T) | 76 | 66 | 90 | 0.86 |
| **Testing set A+B - Stage II-IV only** (30 N, 32 T) | 84 | 78 | 90 | 0.82 |

| | | | | |
|---|---|---|---|---|
| **Table 2** Performances of the 34-miRNA predictor model. The overall accuracy (ACC) is shown together with the sensitivity (SEN, the probability for a tumor to be correctly predicted as "tumor"), specificity (SPE, the probability for a normal sample to be correctly predicted as "normal") and area under curve (AUC, from receiver operating characteristic curves, see Fig. 2A). ^{§}, Accuracy, Sensitivity and Specificity in the training set are based on K-fold (K=5) cross-validation result of the DLDA classifier (see Method). N*, since in the testing set B "normal" sera were not present, ACC, SEN, SPE and AUC were derived considering sera from the testing set B and normal sera from the testing set A. N, normal; AC, adenocarcinoma; SCC, squamous cell carcinoma; T, tumor. Stage I, lung tumor stage I; Stage II-IV, lung tumor stage II, III and IV. | | | | |

In Table 2, accuracy, sensitivity, specificity of the model are defined in the training test, starting from the total of 147 miRNAs. Accuracy, sensitivity, specificity of the final model are obtained by dividing the training set of 75 patients in 5 parts (K-subsamples) and by using each time 60 patients (union of K-1 subsamples) to select the best miRNA (p-value <0.05) and create the model, and subsequently validate it on the remaining K-subsample (15 patients). All this process is repeated 5 times to ensure that all patients are predicted in the k-subsamples. The results, and thus miRNAs, obtanined from this K-5 fold crossvalidation, are combined and the final accuracy, sensitivity, specificity derived, which is representative of values as obtained in the validation (testing) set. We also repeated the initial subdivision 100 times to avoid that the outcome was influenced by a particular subdivision of the training set in K-subsamples. Finally, in order to validate the *bonafide* of the model, we also exchanged randomly 1000 times the real class of subjects (normal or tumor): the model works only with the set of real subjects (not even one out of a thousand of permutations obtained the same accuracy of the real set). Therefore, the 34miRNA model is generated considering the miRNAs with the best performance among the 147 miRNAs (p-value <0.05).

To further control for the robustness of our findings, authors repeated the evaluation of the performance of the 34-miRNA model using a different method for normalization, the "median" method in which data were normalized based on the median Ct value of all the 147 detected miRNAs (Table 3).

**Table 3. Performance of the predictive model in various sets (median normalization)**

| **SET** | **ACC %** | **SEN %** | **SPE %** | **AUC** |
|---|---|---|---|---|
| **Training set** (39 N, 25 AC) | **78^{§}** | 68^{§} | 85^{§} | 0.87 |
| **Testing set A** (30 N, 22AC, 12 SCC) | 78 | 82 | 73 | 0.88 |
| **Testing set B** (30 N*, 23 AC, 13 SCC) | 70 | 67 | 73 | 0.80 |
| **Testing set A+B** (30 N, 45 AC, 25 SCC) | 74 | 74 | 73 | 0.84 |
| **Testing set A+B - AC only** (30 N, 45 AC) | 72 | 71 | 73 | 0.81 |
| **Testing set A+B - SCC only** (30 N, 25 SCC) | 76 | 80 | 73 | 0.88 |
| **Testing set A+B - Stage I only** (30 N, 38 T) | 75 | 76 | 73 | 0.85 |
| **Testing set A+B - Stage II-IV only** (30 N, 32 T) | 73 | 72 | 73 | 0.82 |

| | | | | |
|---|---|---|---|---|
| **Table 3** Performances of the 34-miRNA predictor model. The overall accuracy (ACC) is shown together with the sensitivity (SEN, the probability for a tumor to be correctly predicted as "tumor"), specificity (SPE, the probability for a normal sample to be correctly predicted as "normal") and area under curve (AUC, from receiver operating characteristic curves, see Fig. 2A). ^{§}, Accuracy, Sensitivity and Specificity in the training set are based on K-fold (K=5) cross-validation result of the DLDA classifier (see Method). N*, since in the testing set B "normal" sera were not present, ACC, SEN, SPE and AUC were derived considering sera from the testing set B and normal sera from the testing set A. N, normal; AC, adenocarcinoma; SCC, squamous cell carcinoma; T, tumor. Stage I, lung tumor stage I; Stage II-IV, lung tumor stage II, III and IV. | | | | |

Differently, Table 4 is generated starting from the 34 microRNAs. Models with 30, 25, 20, 15, 10, 5, 2 and 1 microRNAs (see Fig. 7) were tested in the two cohorts of patients (training and testing sets) and accuracy, sensitivity and specificity are shown in the Table 4.

**Table 4**

| **Training set** (39 N, 25 AC) | | | |
|---|---|---|---|
| **miRNA number** | **ACC %** | **SEN %** | **SPE %** |
| **30** | 84% | 79% | 87% |
| **25** | 84% | 78% | 87% |
| **20** | 84% | 80% | 87% |
| **15** | 84% | 79% | 87% |
| **10** | 82% | 76% | 86% |
| **5** | 76% | 71% | 80% |
| **2** | 71% | 64% | 75% |
| **1** | 64% | 56% | 69% |
| | | | |

| **Testing set** (30 N, 45 AC, 25 SCC) | | | |
|---|---|---|---|
| **miRNA number** | **ACC %** | **SEN %** | **SPE %** |
| **30** | 74% | 68% | 86% |
| **25** | 74% | 68% | 83% |
| **20** | 75% | 71% | 83% |
| **15** | 74% | 74% | 72% |
| **10** | 74% | 70% | 83% |
| **5** | 74% | 72% | 74% |
| **2** | 64% | 62% | 69% |
| **1** | 64% | 62% | 69% |

| | | | |
|---|---|---|---|
| **Table 4.** Performances of the predictor model in the training or testing sets by using 30, or 25, or 20, or 15, or 10, or 5, or 2, or 1 miRNAs (see Fig. 7) to predict presence or absence of lung cancer. The overall accuracy (ACC) is shown together with the sensitivity (SEN, the probability for a tumor sample to be correctly predicted as "tumor"), specificity (SPE, the probability for a normal sample to be correctly predicted as "normal"). N, normal; AC, adenocarcinoma; SCC, squamous cell carcinoma. | | | |

We next performed a series of experiments to assess the clinical utility of the 34-miRNA predictor. We analyzed sera from a group of 33 subjects, who were detected at baseline LD-CT with benign lung nodules and did not develop lung cancer during the entire period of study. This provided us with the opportunity to test whether our predictor could distinguish between benign and frankly malignant lung diseases. We therefore compared the performance of the predictor in the "normal" sera of testing set A and in the sera of the LD-CT-detected benign nodules. Our analysis also included 15 pre-operative sera from subjects with pulmonary hamartomas (PH, from an independent serum collection obtained from subjects who were not LD-CT-screened); these are considered to be benign lesions, although their nosogenesis and malignancy is still controversial, and subjects are recommended for regular follow-up³⁵. There were no significant differences in the average risk of the normal, nodule and PH categories in spite of the fact that the 34-miRNA model and the risk algorithm were derived by training on a dataset (the training set) that did not include nodules or PHs (Fig. 3A).

Next, we analyzed a group of sera collected before the onset of NSCLC (i.e. from subjects who were negative at the screening round but who developed lung cancer >1 year after). For 13 of such cases, we had both the sera harvested before disease onset (BDO), and the tumor sera that were already included in the training or testing sets. When the risk predictor algorithm was applied, it indicated a significantly increased average risk index for sera collected after the onset of the disease (average risk BDO, -7.12; Tumor, 10.36; p <0.001, paired t-test; Fig. 3B). Thus, at least in the cases analyzed, the 34-miRNA model was capable of detecting the conversion from a normal to a malignant state.

Finally, we tackled the question of the specificity of the 34-miRNA predictor for NSCLC detection, as opposed to other types of cancer, by screening sera from a cohort of 18 patients with invasive ductal breast carcinoma and 10 with breast benign nodules. In unsupervised hierarchical clustering, using all 147 miRNAs, the expression profile of these samples did not show major differences with respect to all other sera used in this study (Figure 5). When the 34-miRNA risk predictor algorithm was applied, it could not discriminate between breast tumors and benign breast nodules (p = 0.65; Fig. 3C).

### REFERENCES

1. Veronesi G, Bellomi M, Mulshine JL, et al: Lung cancer screening with low-dose computed tomography: a non-invasive diagnostic protocol for baseline lung nodules. Lung Cancer 61:340-9, 2008
2. Parkin DM, Bray F, Ferlay J, et al: Global cancer statistics, 2002. CA Cancer J Clin 55:74-108, 2005
3. Jemal A, Thun MJ, Ries LA, et al: Annual report to the nation on the status of cancer, 1975-2005, featuring trends in lung cancer, tobacco use, and tobacco control. J Natl Cancer Inst 100:1672-94, 2008
4. Greenlee RT, Murray T, Bolden S, et al: Cancer statistics, 2000. CA Cancer J Clin 50:7-33, 2000
5. Porter JC, Spiro SG: Detection of early lung cancer. Thorax 55 Suppl 1:S56-62, 2000
6. Peto R, Lopez AD, Boreham J, et al: Mortality from smoking worldwide. Br Med Bull 52:12-21, 1996
7. Jemal A, Siegel R, Ward E, et al: Cancer statistics, 2009. CA Cancer J Clin 59:225-49, 2009
8. Henschke Cl, Yankelevitz DF, Libby DM, et al: Survival of patients with stage I lung cancer detected on CT screening. N Engl J Med 355:1763-71,2006
9. Pastorino U, Bellomi M, Landoni C, et al: Early lung-cancer detection with spiral CT and positron emission tomography in heavy smokers: 2-year results. Lancet 362:593-7, 2003
10. Swensen SJ, Jett JR, Hartman TE, et al: Lung cancer screening with CT: Mayo Clinic experience. Radiology 226:756-61, 2003
11. Bach PB, Jett JR, Pastorino U, et al: Computed tomography screening and lung cancer outcomes. JAMA 297:953-61, 2007
12. Chien CR, Chen TH: Mean sojourn time and effectiveness of mortality reduction for lung cancer screening with computed tomography. Int J Cancer 122:2594-9, 2008
13. McMahon PM, Kong CY, Johnson BE, et al: Estimating long-term effectiveness of lung cancer screening in the Mayo CT screening study. Radiology 248:278-87, 2008
14. Reich JM: A critical appraisal of overdiagnosis: estimates of its magnitude and implications for lung cancer screening. Thorax 63:377-83, 2008
15. Welch HG, Woloshin S, Schwartz LM, et al: Overstating the evidence for lung cancer screening: the International Early Lung Cancer Action Program (I-ELCAP) study. Arch Intern Med 167:2289-95, 2007
16. Team NLSTR: The National Lung Screening Trial: Overview and Study Design. Radiology Nov 2. [Epub ahead of print], 2010
17. Tanaka M, Oikawa K, Takanashi M, et al: Down-regulation of miR-92 in human plasma is a novel marker for acute leukemia patients. PLoS One 4:e5532, 2009
18. Lawrie CH: microRNA expression in erythropoiesis and erythroid disorders. Br J Haematol 150:144-51, 2010
19. Skog J, Würdinger T, van Rijn S, et al: Glioblastoma microvesicles transport RNA and proteins that promote tumour growth and provide diagnostic biomarkers. Nature Cell Biology 10:1470-6, 2008
20. Heneghan HM, Miller N, Lowery AJ, et al: Circulating microRNAs as novel minimally invasive biomarkers for breast cancer. Annals of Surgery 251:499-505, 2010
21. Hu Z, Chen X, Zhao Y, et al: Serum microRNA signatures identified in a genome-wide serum microRNA expression profiling predict survival of non-small-cell lung cancer. J Clin Oncol 28:1721-6, 2010
22. Miyachi M, Tsuchiya K, Yoshida H, et al: Circulating muscle-specific microRNA, miR-206, as a potential diagnostic marker for rhabdomyosarcoma. Biochemical and Biophysical Research Communications 400:89-93, 2010
23. Ng EKO, Chong WWS, Jin H, et al: Differential expression of microRNAs in plasma of patients with colorectal cancer: a potential marker for colorectal cancer screening. Gut 58:1375-81, 2009
24. Rabinowits G, Gerçel-Taylor C, Day JM, et al: Exosomal microRNA: a diagnostic marker for lung cancer. Clin Lung Cancer 10:42-6, 2009
25. Resnick KE, Alder H, Hagan JP, et al: The detection of differentially expressed microRNAs from the serum of ovarian cancer patients using a novel real-time PCR platform. Gynecologic Oncology 112:55-9, 2009
26. Tsujiura M, Ichikawa D, Komatsu S, et al: Circulating microRNAs in plasma of patients with gastric cancers. Br J Cancer 102:1174-9, 2010
27. Laterza OF, Lim L, Garrett-Engele PW, et al: Plasma MicroRNAs as sensitive and specific biomarkers of tissue injury. Clinical Chemistry 55:1977-83, 2009
28. Redell JB, Moore AN, Ward lii NH, et al: Human traumatic brain injury alters plasma microRNA levels. Journal of neurotrauma, 2010
29. Rong H, Liu TB, Yang KJ, et al: MicroRNA-134 plasma levels before and after treatment for bipolar mania. Journal of psychiatric research, 2010
30. Wang K, Zhang S, Marzolf B, et al: Circulating microRNAs, potential biomarkers for drug-induced liver injury. Proc Natl Acad Sci USA 106:4402-7, 2009
31. Zhang Y, Jia Y, Zheng R, et al: Plasma MicroRNA-122 as a Biomarker for Viral-, Alcohol-, and Chemical-Related Hepatic Diseases. Clinical Chemistry, 2010
32. Wittmann J, Jack H-M: Serum microRNAs as powerful cancer biomarkers. Biochim Biophys Acta, 2010
33. Schöler N, Langer C, Döhner H, et al: Serum microRNAs as a novel class of biomarkers: a comprehensive review of the literature. Experimental Hematology:1-5, 2010
34. Chin LJ, Slack FJ: A truth serum for cancer-microRNAs have major potential as cancer biomarkers. Cell Res 18:983-4, 2008
35. Guo W, Zhao YP, Jiang YG, et al: Surgical treatment and outcome of pulmonary hamartoma: a retrospective study of 20-year experience. J Exp Clin Cancer Res 27:8, 2008

## Claims

1. A method of diagnosing a lung tumor in a subject that is asymptomatic, or of discriminating between benign and malignant lung nodules in a subject, or of prognosing a lung tumor in a subject, comprising the steps of:
a) detecting at least 5 miRNAs included in the following list of 34 miRNAs, in a biological fluid sample from the subject:
hsa-let-7a (MIMAT0000062, SEQ ID No. 1);
hsa-let-7b (MIMAT0000063, SEQ ID No. 2);
hsa-let-7d (MIMAT0000065, SEQ ID No. 3);
hsa-miR-103 (MIMAT0000101, SEQ ID No. 4);
hsa-miR-126 (MIMAT0000445, SEQ ID No. 5);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-142-3p (MIMAT0000434, SEQ ID No. 9);
hsa-miR-142-5p (MIMAT0000433, SEQ ID No. 10);
hsa-miR-148a (MIMAT0000243, SEQ ID No. 11);
hsa-miR-148b (MIMAT0000759, SEQ ID No. 12);
hsa-miR-17 (MIMAT0000070, SEQ ID No. 13);
hsa-miR-191 (MIMAT0000440, SEQ ID No. 14);
hsa-miR-22 (MIMAT0000077, SEQ ID No. 15);
hsa-miR-223 (MIMAT0000280, SEQ ID No. 16);
hsa-miR-26a (MIMAT0000082, SEQ ID No. 17);
hsa-miR-26b (MIMAT0000083, SEQ ID No. 18);
hsa-miR-28-5p (MIMAT0000085, SEQ ID No. 19);
hsa-miR-29a (MIMAT0000086, SEQ ID No. 20);
hsa-miR-30b (MIMAT0000420, SEQ ID No. 21);
hsa-miR-30c (MIMAT0000244, SEQ ID No. 22);
hsa-miR-32 (MIMAT0000090, SEQ ID No. 23);
hsa-miR-328 (MIMAT0000752, SEQ ID No. 24);
hsa-miR-331-3p (MIMAT0000760, SEQ ID No. 25);
hsa-miR-342-3p (MIMAT0000753, SEQ ID No. 26);
hsa-miR-374a (MIMAT0000727, SEQ ID No. 27);
hsa-miR-376a (MIMAT0000729, SEQ ID No. 28);
hsa-miR-432-staR (MIMAT0002815, SEQ ID No. 29);
hsa-miR-484 (MIMAT0002174, SEQ ID No. 30);
hsa-miR-486-5p (MIMAT0002177, SEQ ID No. 31);
hsa-miR-566 (MIMAT0003230, SEQ ID No. 32);
hsa-miR-92a (MIMAT0000092, SEQ ID No. 33);
hsa-miR-98 (MIMAT0000096, SEQ ID No. 34);
and
b) comparing the amounts of said miRNAs to proper controls, wherein said comparison allows for diagnosing or prognosing a lung tumor, or for discriminating between benign and malignant lung nodules.

2. The method according to claim 1 wherein said at least 5 miRNAs are:
hsa-let-7a (MIMAT0000062, SEQ ID No. 1);
hsa-miR-133b (MIMAT0000770, SEQ ID No. 6);
hsa-miR-139-5p (MIMAT0000250, SEQ ID No. 7);
hsa-miR-342-3p (MIMAT0000753, SEQ ID No. 26);
hsa-miR-486-5p (MIMAT0002177, SEQ ID No. 31).

3. The method according to claim 1 wherein the detecting step a) comprises the detecting of at least 10 miRNAs included in the list of 34 miRNAs as in claim 1.

4. The method according to claim 3 wherein said at least 10 miRNAs are:
hsa-let-7a (MIMAT0000062, SEQ ID No. 1);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-148b (MIMAT0000759, SEQ ID No. 12);
hsa-miR-26b (MIMAT0000083, SEQ ID No. 18);
hsa-miR-32 (MIMAT0000090, SEQ ID No. 23);
hsa-miR-342-3p (MIMAT0000753, SEQ ID No. 26);
hsa-miR-432-staR (MIMAT0002815, SEQ ID No. 29);
hsa-miR-486-5p (MIMAT0002177, SEQ ID No. 31).

5. The method according to claim 1 wherein the detecting step a) comprises the detecting of at least 15 miRNAs included in the list of 34 miRNAs as in claim 1.

6. The method according to claim 5 wherein said at least 15 miRNAs are:
hsa-let-7a (MIMAT0000062, SEQ ID No. 1);
hsa-let-7b (MIMAT0000063, SEQ ID No. 2);
hsa-let-7d (MIMAT0000065, SEQ ID No. 3);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-148b (MIMAT0000759, SEQ ID No. 12);
hsa-miR-17 (MIMAT0000070, SEQ ID No. 13);
hsa-miR-26b (MIMAT0000083, SEQ ID No. 18);
hsa-miR-29a (MIMAT0000086, SEQ ID No. 20);
hsa-miR-32 (MIMAT0000090, SEQ ID No. 23);
hsa-miR-342-3p (MIMAT0000753, SEQ ID No. 26);
hsa-miR-432-staR (MIMAT0002815, SEQ ID No. 29);
hsa-miR-486-5p (MIMAT0002177, SEQ ID No. 31);
hsa-miR-92a (MIMAT0000092, SEQ ID No. 33).

7. The method according to claim 1 wherein the detecting step a) comprises the detecting of at least 20 miRNAs included in the list of 34 miRNAs as in claim 1.

8. The method according to claim 7 wherein said at least 20 miRNAs are:
hsa-let-7a (MIMAT0000062, SEQ ID No. 1);
hsa-let-7b (MIMAT0000063, SEQ ID No. 2);
hsa-let-7d (MIMAT0000065, SEQ ID No. 3);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-148b (MIMAT0000759, SEQ ID No. 12);
hsa-miR-22 (MIMAT0000077, SEQ ID No. 15);
hsa-miR-26a (MIMAT0000082, SEQ ID No. 17);
hsa-miR-26b (MIMAT0000083, SEQ ID No. 18);
hsa-miR-28-5p (MIMAT0000085, SEQ ID No. 19);
hsa-miR-29a (MIMAT0000086, SEQ ID No. 20);
hsa-miR-30b (MIMAT0000420, SEQ ID No. 21);
hsa-miR-32 (MIMAT0000090, SEQ ID No. 23);
hsa-miR-342-3p (MIMAT0000753, SEQ ID No. 26);
hsa-miR-432-staR (MIMAT0002815, SEQ ID No. 29);
hsa-miR-484 (MIMAT0002174, SEQ ID No. 30);
hsa-miR-486-5p (MIMAT0002177, SEQ ID No. 31);
hsa-miR-92a (MIMAT0000092, SEQ ID No. 33);
hsa-miR-98 (MIMAT0000096, SEQ ID No. 34).

9. The method according to claim 1 wherein the detecting step a) comprises the detecting of at least 25 miRNAs included in the list of 34 miRNAs as in claim 1.

10. The method according to claim 9 wherein said at least 25 miRNAs are:
hsa-let-7a (MIMAT0000062, SEQ ID No. 1);
hsa-let-7b (MIMAT0000063, SEQ ID No. 2);
hsa-let-7d (MIMAT0000065, SEQ ID No. 3);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-148b (MIMAT0000759, SEQ ID No. 12);
hsa-miR-17 (MIMAT0000070, SEQ ID No. 13);
hsa-miR-22 (MIMAT0000077, SEQ ID No. 15);
hsa-miR-223 (MIMAT0000280, SEQ ID No. 16);
hsa-miR-26a (MIMAT0000082, SEQ ID No. 17);
hsa-miR-26b (MIMAT0000083, SEQ ID No. 18);
hsa-miR-28-5p (MIMAT0000085, SEQ ID No. 19);
hsa-miR-29a (MIMAT0000086, SEQ ID No. 20);
hsa-miR-30b (MIMAT0000420, SEQ ID No. 21);
hsa-miR-32 (MIMAT0000090, SEQ ID No. 23);
hsa-miR-328 (MIMAT0000752, SEQ ID No. 24);
hsa-miR-342-3p (MIMAT0000753, SEQ ID No. 26);
hsa-miR-374a (MIMAT0000727, SEQ ID No. 27);
hsa-miR-432-staR (MIMAT0002815, SEQ ID No. 29);
hsa-miR-484 (MIMAT0002174, SEQ ID No. 30);
hsa-miR-486-5p (MIMAT0002177, SEQ ID No. 31);
hsa-miR-566 (MIMAT0003230, SEQ ID No. 32);
hsa-miR-92a (MIMAT0000092, SEQ ID No. 33);
hsa-miR-98 (MIMAT0000096, SEQ ID No. 34).

11. The method according to claim 1 wherein the detecting step a) comprises the detecting of at least 30 miRNAs included in the list of 34 miRNAs as in claim 1.

12. The method according to claim 11 wherein said at least 30 miRNAs are:
hsa-let-7a (MIMAT0000062, SEQ ID No. 1);
hsa-let-7b (MIMAT0000063, SEQ ID No. 2);
hsa-let-7d (MIMAT0000065, SEQ ID No. 3);
hsa-miR-103 (MIMAT0000101, SEQ ID No. 4);
hsa-miR-126 (MIMAT0000445, SEQ ID No. 5);
hsa-miR-133b (MIMAT0000770, SEQ ID No. 6);
hsa-miR-139-5p (MIMAT0000250, SEQ ID No. 7);
hsa-miR-140-5p (MIMAT0000431, SEQ ID No. 8);
hsa-miR-148b (MIMAT0000759, SEQ ID No. 12);
hsa-miR-17 (MIMAT0000070, SEQ ID No. 13);
hsa-miR-191 (MIMAT0000440, SEQ ID No. 14);
hsa-miR-22 (MIMAT0000077, SEQ ID No. 15);
hsa-miR-223 (MIMAT0000280, SEQ ID No. 16);
hsa-miR-26a (MIMAT0000082, SEQ ID No. 17);
hsa-miR-26b (MIMAT0000083, SEQ ID No. 18);
hsa-miR-28-5p (MIMAT0000085, SEQ ID No. 19);
hsa-miR-29a (MIMAT0000086, SEQ ID No. 20);
hsa-miR-30b (MIMAT0000420, SEQ ID No. 21);
hsa-miR-30c (MIMAT0000244, SEQ ID No. 22);
hsa-miR-32 (MIMAT0000090, SEQ ID No. 23);
hsa-miR-328 (MIMAT0000752, SEQ ID No. 24);
hsa-miR-342-3p (MIMAT0000753, SEQ ID No. 26);
hsa-miR-374a (MIMAT0000727, SEQ ID No. 27);
hsa-miR-376a (MIMAT0000729, SEQ ID No. 28);
hsa-miR-432-staR (MIMAT0002815, SEQ ID No. 29);
hsa-miR-484 (MIMAT0002174, SEQ ID No. 30);
hsa-miR-486-5p (MIMAT0002177, SEQ ID No. 31);
hsa-miR-566 (MIMAT0003230, SEQ ID No. 32);
hsa-miR-92a (MIMAT0000092, SEQ ID No. 33);
hsa-miR-98 (MIMAT0000096, SEQ ID No. 34).

13. The method according to claim 1 wherein the detecting step a) comprises the detecting of all miRNAs included in the list of 34 miRNAs as in claim 1.

14. The method according to any of previous claims wherein the biological fluid sample is comprised in the group of: blood, serum, plasma, urine, saliva, mucus, tears, amniotic fluid, breast milk, sputum, cerebrospinal fluid, peritoneal fluid, pleural fluid and seminal fluid, or fractions thereof.

15. The method according to any of previous claims wherein the lung tumor is a non-small cell lung carcinoma (NSCLC).

16. The method according to any of previous claims wherein the detection of said miRNAs is performed by means of hybridization with primers and/or probes, each one specific and selective for the sequence of one of miRNAs.

17. The method according to any of previous claims 1 to 16 wherein the detection of said miRNAs is performed by microarray or sequencing.

18. The method according to any of previous claims wherein proper controls comprise same miRNAs from healthy subjects.

19. The method according to any of previous claims comprising the step of normalizing the amounts of said miRNAs with at least one unrelated miRNA present in the same biological fluid sample, and/or with at least one unrelated miRNA exogenously spiked in the sample.

20. The method according to claim 19 wherein said at least one unrelated miRNA belongs to the group of miR-197, SEQ ID No. 35; miR-19b, SEQ ID No. 36; miR-24, SEQ ID No. 37; miR-146a, SEQ ID No. 38; miR-15b, SEQ ID No. 39; miR-19a, SEQ ID No. 40.

21. The method according to claim 20 wherein the said unrelated miRNAs are miR-197, SEQ ID No. 35; miR-19b, SEQ ID No. 36; miR-24, SEQ ID No. 37; miR-146a, SEQ ID No. 38; miR-15b, SEQ ID No. 39; miR-19a, SEQ ID No. 40.

22. The method according to any of previous claims 1-19 comprising the step of normalizing the amounts of miRNAs according to claims 1 to 13 with the median of the amount of all miRNAs present in the same biological fluid sample.

23. A method of monitoring a lung tumor status after treatment with surgery and/or chemotherapy and/or radiotherapy in a subject with said lung tumor, comprising the step of following the modulation of miRNAs as claimed in any of previous claims.

24. A kit to perform the method according to any of previous claims comprising specific primers and/or probes for each of miRNAs to be detected.

25. A microarray or a polymerase chain reaction (PCR) or a sequencing based plate to perform the method according to any of previous claims comprising specific probes for each of miRNAs to be detected.
